(19) Europäisches Patentamt
European Patent Office
Office européen des brevets

(11) Veröffentlichungsnummer: **0 305 743 B1**

(12) # EUROPÄISCHE PATENTSCHRIFT

(45) Veröffentlichungstag der Patentschrift: **04.11.92**

(51) Int. Cl.$^5$: **A61K 31/725**, //(A61K31/725, 31:52)

(21) Anmeldenummer: **88112332.7**

(22) Anmeldetag: **29.07.88**

(54) **Pharmazeutisches Kombinationspräparat sowie dessen Herstellung und Verwendung.**

(30) Priorität: **01.08.87 DE 3725554**

(43) Veröffentlichungstag der Anmeldung:
**08.03.89 Patentblatt 89/10**

(45) Bekanntmachung des Hinweises auf die Patenterteilung:
**04.11.92 Patentblatt 92/45**

(84) Benannte Vertragsstaaten:
**AT BE CH DE ES FR GB GR IT LI LU NL SE**

(56) Entgegenhaltungen:
**EP-A- 0 270 317**
**DE-A- 2 364 373**

(73) Patentinhaber: **HOECHST AKTIENGESELLSCHAFT**
**Postfach 80 03 20**
**W-6230 Frankfurt am Main 80(DE)**

(72) Erfinder: **Schrinner, Elmar, Dr.**
**Hedwigstrasse 2**
**W-6200 Wiesbaden(DE)**
Erfinder: **Winkler, Irvin, Dr.**
**In den Eichen 40**
**W-6237 Liederbach(DE)**
Erfinder: **Meichsner, Christoph, Dr.**
**Bienerstrasse 30**
**W-6238 Hofheim am Taunus(DE)**
Erfinder: **Helsberg, Matthias, Dr.**
**Münsterer Strasse 14**
**W-6233 Kelkheim (Taunus)(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die gezielte Suche nach antiviral wirksamen Substanzen setzt die Existenz virusspezifischer Stoffwechselvorgänge im infizierten Organismus voraus. Die Retroviren und mit ihnen HIV (Human Immuno Deficiency Virus) benötigen im Verlauf des Infektionszyklus die Aktivität einer RNA-abhängigen DNA-Polymerase, um ihre in Form einer RNA codierte Erbinformation in eine DNA umzuschreiben, die in das Wirtsgenom integriert und von entsprechenden Wirtsenzymen in wirksame Genprodukte umgesetzt werden kann. Das reverse Transkriptase (RT) genannte Enzym ist Bestandteil des infizierenden Viruspartikels und findet sich normalerweise nicht im menschlichen oder tierischen Organismus. Die RT erscheint deshalb als geeigneter Angriffspunkt einer Chemotherapie von Retroviruserkrankungen und somit auch des Acquired Immuno Deficiency Sydroms (AIDS), das die späte Folge einer Infektion durch das HIV darstellt.

Es ist bekannt, daß sulfatierte Polysaccharide die Vermehrung von Retroviren in der Zellkultur hemmen können (Klement & Nicolson in Mormorosch & Koprowski, Methods in Virology VI, (1977) 60-103). Diese Stoffe sind hochwirksame Inhibitoren der RT. Eine dieser Substanzen ist z.B. Pentosanpolysulfat - ein 1,4-verknüpftes Pyranose-Polymer mit bis zu 2 Schwefelsäureestergruppen pro Zuckereinheit; d.h. eine Verbindung mit folgender Formel:

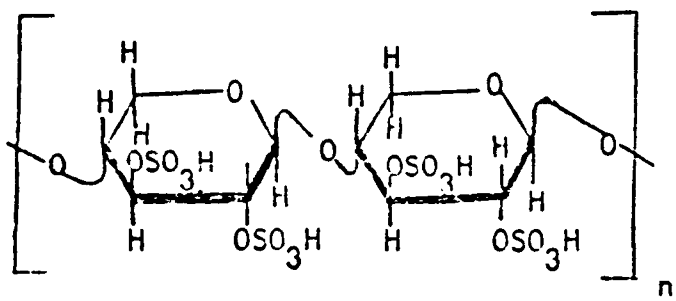

n = Anzahl der Struktureinheiten

Nebenwirkungen dieser Verbindung sind u.a. Blutgerinnungshemmung und Störung des Haarwachstums.

Es ist eine Reihe von weiteren Substanzen bekannt, die verschiedene Schritte der durch die RT katalysierten Reaktion hemmen (De Clerq & Balzarini: Antiviral Research Suppl. 1 (1985) 89-94). Unter diesen wurden bisher in klinischen Prüfungen u.a. Suramin, eine Verbindung der Formel

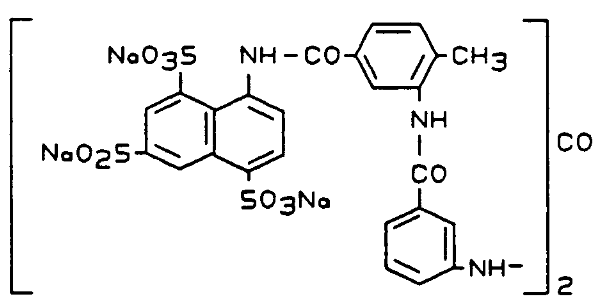

,

Polyantimonwolframsäure ($(NH_4)_{18}(NaW_{21}Sb_9O_{86})_{17}$), und Phosphonoameisensäure ($(OH)_2(O)PCOOH$) mit geringem Erfolg eingesetzt. Erst das synthetische Nukleosid 3′-Azido-3′-desoxythymidin (AZT), eine Verbindung der Formel

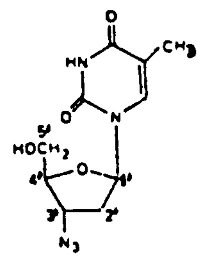

erbrachte gesicherte therapeutische Erfolge in der klinischen Prüfung (Yarchoan et al.: The Lancet 575-580 (1986)), ist aber mit dem Makel schwerwiegender Nebenwirkungen behaftet, die eine nach heutigen Erkenntnissen notwendige Dauertherapie nicht zulassen.

Überraschenderweise wurde nun gefunden, daß die Wirksamkeit der sulfatierten Polysaccharide durch die gleichzeitige Verabreichung mindestens eines Xanthinderivats deutlich erhöht werden kann.

Es sind zwar aus der DE OS 2364373 pharmazeutische Präparate bekannt, die u.a. Xanthinderivate und ein sulfatiertes Polysaccharid enthalten, die Kombinationen der genannten Stoffe in der DE OS unterscheiden sich aber von denen der vorliegenden Anmeldung und werden für eine andere Indikation (Aknebehandlung etc.) beschrieben.

Xanthin ist die Verbindung der Formel:

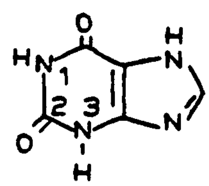

Durch die Kombination mit Xanthinderivaten können die sulfatierten Polysaccharide in geringerer Menge als ohne die genannten Xanthinderivate verabreicht werden, um die gleiche Wirkung zu erzielen. Eine derartige Darreichungsform bat den Vorteil, daß die Nebenwirkungen des sulfatierten Polysaccharids entsprechend geringer sind. Die genannte Darreichungsform ist für eine Dauertherapie geeignet.

Gegenstand der Erfindung ist demzufolge ein pharmazeutisches Kombinationspräparat, das dadurch gekennzeichnet ist, daß es als Komponente

a) mindestens ein sulfatiertes Polysaccharid ausgewählt aus Dextranpolysulfat, Leavanpolysulfat und Pentosanpolysulfat, insbesondere Pentosanpolysulfat und

b) mindestens ein Xanthinderivat der Gruppe der folgenden Verbindungen:

b1) Verbindungen der Formel I

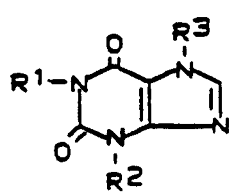

( I )

in der einer der Reste $R^1$ und $R^3$ eine geradkettige Alkyl-, ($\omega$-1)-Oxoalkyl- oder ($\omega$-1)-Hydroxyalkylgruppe mit 3 bis 8 C-Atomen und die beiden anderen Reste $R^2$ und $R^3$ oder $R^1$ und $R^2$ geradkettige oder verzweigte Alkylgruppen mit 1 bis 8 C-Atomen in der Position von $R^1$ und $R^3$ und von 1 bis 4 C-Atomen in der Position von $R^2$ darstellen, wobei die Summe der C-Atome dieser beiden Alkylsubstituenten höchstens 10 beträgt,

b2) Verbindungen der Formel II

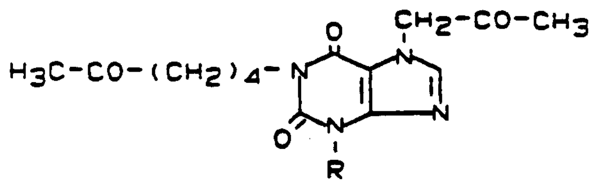

(II)

in der R für einen Alkylrest mit 1 bis 4 C-Atomen steht,
b3) Verbindungen der Formel III

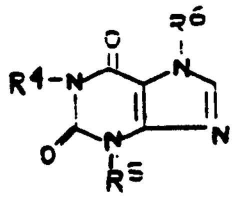

(III),

in der mindestens einer der Reste $R^4$ und $R^6$ eine tertiäre Hydroxyalkylgruppe der Formel

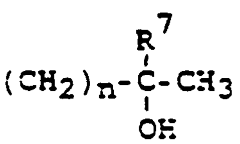

(IIIa)

darstellt, wobei $R^7$ eine Alkylgruppe mit bis zu 3 C-Atomen und n eine ganze Zahl von 2 bis 5 bedeuten, und - falls nur einer der Reste $R^4$ oder $R^6$ eine solche tertiäre Hydroxyalkylgruppe der Formel IIIa bedeutet - der andere Rest für ein Wasserstoffatom oder einen aliphatischen Kohlenwasserstoff-Rest $R^8$ mit bis zu 6 C-Atomen steht, dessen Kohlenstoffkette von bis zu 2 Sauerstoffatomen unterbrochen oder mit einer Oxogruppe oder bis zu zwei Hydroxygruppen substituiert sein kann, und $R^5$ eine Alkylgruppe mit 1 bis 4 C-Atomen darstellt, enthält oder aus diesen Komponenten besteht.

Das sulfatierte Polysaccharid kann hierbei als freie Säure, d.h. mit der Gruppierung $O-SO_3H$, oder als physiologisch verträgliches Salz, vorzugsweise als Ammonium- oder Alkalisalz, d.h. mit der Gruppierung $O-SO_3A$ (A = Ammonium oder Alkali), vorliegen.

Das mittlere Molekulargewicht der sulfatierten Polysaccharide kann einen weiten Bereich überstreichen. Bevorzugt ist ein Bereich von 1000 bis 200000 Dalton, besonders bevorzugt von 5000 bis 12000 Dalton, insbesondere von 6000 Dalton.

Der Sulfatierungsgrad der Polysaccharid-OH-Gruppen, d.h. die durchschnittliche Anzahl von Sulfatresten pro Monosaccharideinheit, kann in weiten Grenzen variiert werden. Vorzugsweise liegt der Sulfatierungsgrad der Polysaccharid-OH-Gruppen in einem Bereich von 20 bis 100 % der vorhandenen OH-Gruppen, besonders bevorzugt von 80 bis 95 %, insbesondere von 90 %.

Von den Xanthinderivaten sind wiederum jene der Formel I besonders bevorzugt, die in der Position von $R^1$ oder $R^3$ eine Hexyl-, 5-Oxohexyl- oder 5-Hydroxyhexylgruppe tragen. Zu ihnen gehören insbesondere
1-Hexyl-3,7-dimethylxanthin,
1-(5-Hydroxyhexyl)-3,7-dimethylxanthin,
3,7-Dimethyl-1-(5-oxohexyl)-xanthin,
7-(5-Hydroxyhexyl)-1,3-dimethylxanthin,
1,3-Dimethyl-7-(5-oxohexyl)-xanthin,
1-(5-Hydroxyhexyl)-3-methyl-7-propylxanthin und 3-Methyl-1-(5-oxohexyl)-7-propylxanthin,
vor allem 3,7-Dimethyl-1-(5-Oxohexyl)-xanthin (= Pentoxyfyllin).

Besonders bevorzugte Verbindungen der Formel III sind solche Verbindungen, bei denen $R^5$ für eine Methyl- oder Ethylgruppe steht. Gleichermaßen bevorzugt sind jene Verbindungen der Formel III, in denen

nur einer der beiden Reste $R^4$ oder $R^6$ die vorstehend definierte tertiäre Hydroxyalkylgruppe darstellt. Weiterhin bevorzugt sind solche Verbindungen, bei denen $R^7$ für eine Methylgruppe steht und n eine ganze Zahl von 3 bis 5 bedeutet, so daß der tertiäre Hydroxyalkylrest IIIa entweder [($\omega$-1)-Hydroxy-($\omega$-1)-methyl]-pentyl, -hexyl oder -heptyl darstellt, insbesondere solche, bei denen $R^5$ Methyl oder Ethyl bedeutet.

Weiterhin sind diejenigen Verbindungen der Formel III besonders hervorzuheben, in denen $R^4$ die tertiäre Hydroxyalkylgruppe darstellt und $R^6$ für Alkyl, Hydroxyalkyl oder Alkoxyalkyl mit jeweils 1 bis 4 C-Atomen steht, wie etwa das 7-Ethoxymethyl-1-(5-hydroxy-5-methylhexyl)-3-methylxanthin.

Eine weitere Ausführungsform der Erfindung besteht darin, daß die Oxoalkylxanthine der Formeln I und II bzw. die Hydroxyalkylxanthine der Formel I und III nicht per se, sondern in Prodrug-Form eingesetzt werden, aus der erst im Organismus die therapeutisch wirksamen Xanthinverbindungen mit den in Formeln I, II und III definierten Substituenten durch Biotransformation freigesetzt werden können. Hierfür kommen beispielsweise die acetalisierten Oxoalkylxanthine, in denen die Carbonylgruppen durch das Strukturelement der Formel IV

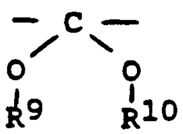

(IV)

ersetzt sind, und die O-acylierten Hydroxyalkylxanthine mit dem Strukturelement der Formel (V)

$R^{11}$-CO-O-    (V)

anstelle der Hydroxyfunktion in Frage, wobei $R^9$ und $R^{10}$ jeweils eine Alkylgruppe mit bis zu 4 C-Atomen oder zusammen eine Ethylen-, Trimethylen- oder Tetramethylengruppe darstellen und $R^{11}$ einen Alkylrest mit bis zu 4 C-Atomen oder gegebenenfalls substituiertes Phenyl oder Pyridyl bedeutet.

Das Gewichtsverhältnis der Komponente a) zur Komponente b) der erfindungsgemäßen Kombinationspräparate kann einen weiten Bereich überstreichen. Zu bevorzugen ist ein Gewichtsverhältnis der Komponente a) zur Komponente b) von 1:100 bis 100:1, besonders bevorzugt von 1:10 bis 30:1.

Zur Behandlung bzw. zur Prophylaxe von Erkrankungen, die durch Viren - insbesondere durch Retroviren - verursacht werden, können die erfindungsgemäßen Kombinationspräparate auf unterschiedliche Weise verabreicht werden. Z.B. können sie intravenös, intramuskulär, intraperitoneal, subkutan, als Dauertropfinfusion oder oral verabreicht werden. Bei akuten Krankheitszuständen ist die Verabreichung als Dauertropfinfusion vorzuziehen. Zur Dauermedikation ist die orale Verabreichung angezeigt.

Die Herstellung der erfindungsgemäßen Kombinationspräparate erfolgt, indem mindestens ein sulfatiertes Polysaccharid und mindestens ein Xanthinderivat gegebenenfalls mit weiteren Zusatz- und/oder Hilfsstoffen in eine geeignete Darreichungsform gebracht werden. Die Zusatz- oder Hilfsstoffe stammen aus der Gruppe der Trägermittel, Konservierungsstoffe und anderer üblicher Hilfsstoffe. Z.B. können für orale Darreichungsformen Hilfsstoffe wie Stärke, z.B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z.B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente des Kombinationspräparats, ein Retardierungsmittel zuzusetzen, gegebenenfalls in Form von permeablen Membranen, wie z.B. solche auf Cellulose- oder Polystyrolharzbasis, oder Ionenaustauscher.

Die anzuwendende Dosierung der erfindungsgemäßen Kombinationspräparate ist abhängig von verschiedenen Faktoren wie Darreichungsform des Medikaments und Zustand und Gewicht des Patienten. Eine Tagesdosis von 800 mg sulfatiertem Polysaccharid und 800 mg eines Xanthinderivats sollte jedoch nur kurzzeitig überschritten werden. Bevorzugt sind 100 bis 600 mg sulfatiertes Polysaccharid und 75 bis 600 mg Xanthinderivat jeweils als Tagesdosis. Darüber hinaus ist es zweckmäßig, für einzelne konkrete Wirkstoffkombinationen sowohl Zusammensetzung als auch Dosierung in Versuchen zu testen. Diese Versuche können in vitro (vgl. z.B. den nachfolgend beschriebenen Wirksamkeitstest I) und auch in vivo (vgl. z.B. nachfolgend beschriebenen Wirksamkeitstest II) durchgeführt werden.

Die für die erfindungsgemäßen Kombinationspräparate notwendigen Wirkstoffe lassen sich auf folgende Weise herstellen:

1. Sulfatierte Polysaccharide der Komponente a):

Die sulfatierten Polysaccharide, die teilweise auch Handelspräparate sind (z.B. Pentosanpolysulfat SP 54®, mittleres Molekulargewicht ca. 6000 Dalton, Sulfatierungsgrad ca. 90 %, Hersteller: bene-Chemie GmbH, 8000 München 71, Herterichstraße 1), lassen sich z.B. auf folgende Weise herstellen (R.L. Whistler, Meth. Carbohydr. Chem. IV, 426-429 (1972)): zunächst wird ein DMSO-Schwefeltrioxid-Komplex hergestellt, indem Schwefeltrioxid bei ca. 15 °C bis 17 °C in die ca. 4-fache Volumenmenge DMSO unter Rühren eingetropft wird. Anschließend wird ein Lösungsmittel, vorzugsweise Methylenchlorid, (vorzugsweise das 4-fache des Volumens des Schwefeltrioxids), zugegeben. Der anfallende feste Komplex läßt sich durch Filtration abtrennen und kann anschließend getrocknet werden. Das zu sulfatierende Polysaccharid (erhältlich z.B. nach Meth. Carbohydr. Chem. IV (1972) [1]-[4], [7]) wird in einem Lösungsmittel, z.B. DMSO, gelöst und bei einer Temperatur von vorzugsweise ca. 15 °C bis 17 °C mit dem wie oben beschrieben gewonnenen Komplex versetzt. Die für einen bestimmten Sulfatierungsgrad nötige Menge an Komplex ist für jedes Polysaccharid empirisch zu bestimmen. Nachdem Eiswasser zugegeben und die Lösung mit Alkalihydroxyd neutralisiert wurde, kann das sulfatierte Produkt z.B. durch Zugabe von Methanol ausgefällt werden. Das erhaltene Produkt ist zweckmäßigerweise weiter zu reinigen; besonders vorteilhafte Reinigungsverfahren sind die chromatographische Trennung und die Gelfiltration, was auch eine Trennung in Abhängigkeit vom Sulfatierungsgrad und Molgewicht zuläßt.

2. Xanthinderivate der Komponente b):

Die Herstellung der Xanthinderivate, die teilweise ebenfalls Handelsprodukte sind (z.B. ®Trental, Wirkstoff Pentoxifyllin, Firma Hoechst AG, Frankfurt), kann z.B. erfolgen wie dies für verschiedene konkrete Verbindungen in der DE-B 1 233 405 oder DE-B 1 235 320 beschrieben ist.

Durch die nachfolgenden Zubereitungsbeispiele soll die Erfindung näher erläutert werden.

**Beispiel 1:**

Tabletten, die für die orale Verabreichung geeignet sind und die nachfolgenden Bestandteile enthalten, werden auf an sich bekannte Weise hergestellt, indem man Wirk- und Hilfsstoffe granuliert und anschließend zu Tabletten verpreßt. Diese Tabletten eignen sich zur antiviralen Behandlung in einer Dosis von einer Tablette 3-4 mal täglich.

| Bestandteile (pro Tabelette) | Gewicht |
|---|---|
| Pentosanpolysulfat, Na-Salz Mittleres Molekulargew. 6000 Dalton Sulfatierungsgrad 90 % | 100 mg |
| Pentoxifyllin | 50 mg |
| Milchzucker | 150 mg |
| Maisstärke | 50 mg |
| Talkum | 6 mg |
| kolloidales Siliziumdioxid | 6 mg |
| Magnesiumstearat | 4 mg |

**Beispiel 2**

Kapseln, die für die orale Verabreichung geeignet sind, enthalten die nachfolgend genannten Bestandteile und können auf an sich bekannte Weise hergestellt werden, indem man Wirk- und Hilfsstoffe vermischt und in Gelantinekapseln abfüllt. Diese Kapseln dienen zur antiviralen Behandlung in einer Dosis von einer Kapsel 2-4 mal täglich.

| Bestandteile (pro Kapsel) | Gewicht |
|---|---|
| Pentosanpolysulfat, Na-Salz mittleres Molekulargewicht 6000 Dalton, Sulfatierungsgrad 90 % | 150 mg |
| Pentoxifyllin | 75 mg |
| Milchzucker | 200 mg |
| Talkum | 10 mg |
| kolloidales Siliziumdioxid | 10 mg |

**Beispiel 3**

Wirkstofflösungen, die zur Injektion geeignet sind, enthalten die nachfolgend genannten Bestandteile und können auf an sich bekannte Weise hergestellt werden, indem die Stoffe miteinander vermischt und in sterile Ampullen abgefüllt werden, die mit einer Gummikappe verschlossen werden. Die Injektionslösungen dienen zur antiviralen Behandlung in einer Dosis von 1-2 Injektionseinheiten (1 Injektionseinheit = 1 Ampulle) pro Tag.

| Bestandteile (pro Ampulle) | Gewicht |
|---|---|
| Pentosanpolysulfat, Na-Salz mittleres Molekulargewicht 6000 Dalton, Sulfatierungsgrad 90 % | 150 mg |
| Pentoxifyllin | 25 mg |
| Natriumchlorid | 50 mg |
| Methylparaben | 5 mg |
| steriles Wasser | 5 g |

**Wirksamkeitstests**

I) Wirksamkeit gegen Human Immuno Deficiency Virus (HIV) in der Zellkultur

Zur Prüfung der Hemmwirkung von verschiedenen Kombinationspräparaten auf die Replikation von HIV wurden Lymphozyten aus frischem Humanblut durch Gradientenzentrifugation isoliert, durch Zugabe von Phytohämagglutinin (Wirkstoff auf N-Acetyl-Galactosamin-Basis, gewonnen aus Phaseolus vulgaris) in RPMI 1640-Medium (Zellzüchtungssubstrat, Mischung aus Aminosäuren, Vitaminen, Mineralstoffen und Puffersubstanzen, vgl. G.E. Moore et. al. J.A.M.A. 199, 519 (1967)) mit 10 % fötalem Kälberserum stimuliert und anschließend in komplettem RPMI-Medium (komplettes RPMI-Medium = RPMI 1640 + Kälberserum) mit 20 i.E./ml rekombinantem Interleukin 2 (Kulitz et al. Drug Res. 35, 1607 (1985)) drei Tage bei 37 °C kultiviert. Die stimulierten Lymphozyten wurden in Einzelportionen abgefüllt und in flüssigem Stickstoff gelagert. Für die Versuche wurden Zellen aufgetaut und drei bis vier Tage in komplettem RPMI-Medium mit rekombinantem Interleukin 2 kultivert. Zur Infektion wurden die Zellen mit dem zellfreien Überstand HIV-infizierter Zellkulturen versetzt und 30 Minuten inkubiert. Nach einmaligem Waschen wurden die infizierten Zellen in komplettem RPMI-Medium mit Interleukin 2 aufgenommen, dem die Prüfsubstanz in entsprechender Konzentration zugesetzt war, und in 24er Multiwellschalen eingesät. Nach 3 Tagen Inkubation bei 37 °C wurde jeweils die Hälfte des Mediums entfernt und durch frisches, wirkstoffhaltiges Medium ersetzt. Am vierten Tag nach der Infektion erfolgte die Auswertung durch mikroskopische Begutachtung der Zellkulturen. Der Grad der Virusvermehrung wurde anhand der Anzahl und Größe der Syncytien abgeschätzt. In Tabelle 1 ist die Konzentration der jeweiligen Wirkstoffe eingetragen, und es wird für jede Zusammensetzung vermerkt, ob eine Hemmung der Syncytienbildung zu beobachten ist oder nicht. Wie Tabelle 1 zeigt, läßt sich mit allen untersuchten sulfatierten Polysacchariden eine Hemmung der Syncytienbildung erreichen, indem einer bei alleiniger Verabreichung unwirksamen Menge an sulfatiertem Polysaccharid eine bestimmte Menge an Xanthinderivat zugesetzt wird. Die Wirksamkeitssteigerung der sulfatierten Polysaccharide durch Zusatz von Xanthinderivat wird hierdurch nachgewiesen.

Tabelle 1

| Komponente A | Konzentration µg/ml | Komponente B | Konzentration µg/ml | Hemmung der Syncytienbildung |
|---|---|---|---|---|
| Laevanpoly- | 10 | Pentoxi- | - | nein |
| sulfat, | 20 | fyllin | - | nein |
| Na-Salz, | 50 | | - | ja |
| mittleres | | | | |
| Molekulargew. | 10 | | 5 | nein |
| 8000 Dalton, | 10 | | 25 | nein |
| Sulfatierungs- | 10 | | 100 | nein |
| grad 100 % | | | | |
| Laevanpoly- | 25 | Pentoxi- | 5 | nein |
| sulfat, | 25 | fyllin | 25 | ja |
| Na-Salz, | 25 | | 100 | ja |
| mittleres | - | | 100 | nein |
| Molekulargew. | | | | |
| 8000 Dalton, | | | | |
| Sulfatierungs- | | | | |
| grad 100 % | | | | |

**Fortsetzung der Tabelle 1**

| Komponente A | Konzentration µg/ml | Komponente B | Konzentration µg/ml | Hemmung der Syncytienbildung |
|---|---|---|---|---|
| Dextranpoly- | 10 | Pentoxi- | 5 | nein |
| sulfat, | 10 | fyllin | 25 | nein |
| Na-Salz, | 10 | | 100 | nein |
| mittleres | | | | |
| Molekulargew. | 25 | | 5 | nein |
| 8000 Dalton, | 25 | | 25 | nein |
| Sulfatierungs- | 25 | | 100 | ja |
| grad 80 % | - | | 100 | nein |
| Pentosanpoly- | 10 | 1-(5-Hydroxy- | 5 | nein |
| sulfat SP 54® | 10 | hexyl)-3-methyl- | 25 | nein |
| Na-Salz, | 10 | 7-propyl-xanthin | 100 | ja |
| mittleres | - | | 100 | nein |
| Molekulargew. | | | | |
| 6000 Dalton, | | | | |
| Sulfatierungs- | | | | |
| grad 90 % | | | | |
| Pentosanpoly- | - | Pentoxi- | 100 | nein |
| sulfat SP 54® | 5 | fyllin | - | nein |
| Na-Salz, | 5 | | 1 | nein |
| mittleres | 5 | | 10 | nein |
| Molekulargew. | 5 | | 100 | nein |
| 6000 Dalton, | | | | |
| Sulfatierungs- | 10 | | - | nein |
| grad 90 % | 10 | | 1 | nein |
| | 10 | | 10 | ja |
| | 10 | | 100 | ja |
| Pentosanpoly- | 25 | Pentoxi- | - | ja |
| sulfat SP 54® | 25 | fyllin | 1 | ja |
| Na-Salz, | 25 | | 10 | ja |
| mittleres | 25 | | 100 | ja |
| Molekulargew. | - | | 100 | nein |
| 6000 Dalton, | | | | |
| Sulfatierungs- | | | | |
| grad 90 % | | | | |

II) Wirksamkeit von Pentosanpolysulfat (SP 54)® gegen Retrovirusinfektionen in der Maus

Da für die HIV-Infektion des Menschen kein geeignetes Infektionsmodell bei Labortieren existiert, muß bei der Prüfung von Chemotherapeutika auf Infektionen mit anderen Retroviren zurückgegriffen werden. Sowie die Hemmung der Virusreplikation in vivo aufgrund einer spezifischen Blockierung der RT-Aktivität untersucht wird, erscheint die Wahl eines solchen Ersatzmodelles gerechtfertigt. Im vorliegenden Fall wurde

die Infektion der Maus mit dem Friend-Leukämie-Virus gewählt.

Dazu wurden normale Labormäuse (NMRI = Naval Medical Research Institute) durch intravenöse Injektion mit Friend-Virus enthaltendem Mäuseserum infiziert. Bei den unbehandelten Kontrolltieren entwikkelte sich als Symptom der Infektion innerhalb von zwei Wochen eine deutliche Vergrößerung von Milz und Leber. Die Behandlung erfolgte über 10 Tage, beginnend 48 Stunden nach der Infektion. Am 14. Versuchstag wurden die Tiere durch Luxation der Halswirbel getötet und geöffnet. Die Milz wurde entnommen und gewogen. Als Meßparameter der therapeutischen Wirksamkeit wurde das Milzgewicht der behandelten Tiere mit dem der unbehandelten Infektionskontrolle in Relation gesetzt.

Während bei uninfizierten ausgewachsenen Labormäusen (20-24 g Köpergewicht) die Milz weniger als 1 % des Körpergewichts wog, erreichte bei infizierten Tieren die Milz am Ende des Versuchs etwa 10 % des Körpergewichts. Die Behandlung mit Suramin führte zu einer Verringerung des Milzwachstums, die statistisch am meisten gesichert werden konnte ($P < 0,05$). Auch Pentosanpolysulfat, allein verabreicht, zeigte einen deutlichen Effekt. Dextranpolysulfat, allein verabreicht und in Kombination mit Pentoxifyllin, war in dem untersuchten Tiermodell ebenfalls wirksam, wobei wegen der relativ geringen Tierzahlen ein Vergleich mit Pentosanpolysulfat nicht möglich war. Die kombinierte Gabe von Pentosanpolysulfat und Pentoxifyllin verringerte den Grad der Splenomegalie (Milzschwellung) gegenüber der Behandlung mit Pentosanpolysulfat allein. Pentoxifyllin übte keinen Einfluß auf den Infektionsverlauf aus.

Die relativ hohe Sterblichkeit der Mäuse bei der Applikation von Pentosanpolysulfat war auf Blutungen infolge der Stichverletzungen bei der Injektion der Medikamte zurückzuführen, was bei kleineren Versuchstieren häufig auftritt. Bei größeren Versuchstieren bzw. bei Menschen ist die Gefahr des Verblutens infolge einer Stichverletzung durch eine Injektionsnadel zu vernachlässigen.

Tabelle 2

Therapeutische Wirkung von Pentosanpolysulfat (SP 54®) in Kombination mit Pentoxifyllin (Trental®) bei der Friend-Virus-Infektion der Maus

| Präparat | Dosierung | rel. Milzgewicht % Körpergewicht | Überlebende/Gruppengröße | Signifikanz |
|---|---|---|---|---|
| Kontrolle | - | 9.22 ± 2.76 | 7/7 | 1.0 |
| Suramin | 10 x 1.0 mg | 4.8 ± 0.96 | 7/7 | < 0,05 |
| Pentosanpolysulfat | 20 x 0.25 mg | 5.67 ± 2.85 | 7/7 | 0.017 |
| Pentosanpolysulfat | 20 x 1.0 mg | 5.88 ± 3.40 | 2/7 | 0.094 |
| Pentoxifyllin | 20 x 0.04 mg | 8.32 ± 1.45 | 7/7 | 0.232 |
| Pentoxifyllin | 20 x 0.4 mg | 9.45 ± 2.0 | 7/7 | 0.428 |
| Pentoxifyllin | 20 x 1.0 mg | 9.08 ± 2.36 | 7/7 | 0.459 |
| Pentosanpolysulfat + Pentoxifyllin | 20 x 0.25 mg<br>20 x 0.04 mg | 5.07 ± 2.1 | 5/7 | 0.009 |
| Pentosanpolysulfat + Pentoxifyllin | 20 x 0.25 mg<br>20 x 0.04 mg | 2.69 ± 1.67 | 3/7 | 0.03 |

**Patentansprüche**

**Patentansprüche für folgende Vertragsstaaten : AT, BE, CH, DE, FR, GB, IT, LI, LU, NL, SE**

**1.** Pharmazeutisches Kombinationspräparat, dadurch gekennzeichnet, daß es die Komponenten

a) mindestens ein sulfatiertes Polysaccharid ausgewählt aus Dextranpolysulfat, Laevanpolysulfat und Pentosanpolysulfat, insbesondere Pentosanpolysulfat und

b) mindestens ein Xanthinderivat der folgenden Verbindungen:

b1) Verbindungen der Formel I

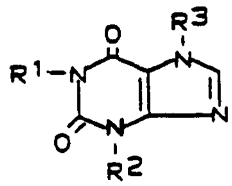

(I)

in der einer der Reste $R^1$ und $R^3$ eine geradkettige Alkyl-, ($\omega$-1)-Oxoalkyl- oder ($\omega$-1)-Hydroxyalkylgruppe mit 3 bis 8 C-Atomen und die beiden anderen Reste $R^2$ und $R^3$ oder $R^1$ und $R^2$ geradkettige oder verzweigte Alkylgruppen mit 1 bis 8 C-Atomen in der Position von $R^1$ und $R^3$ und von 1 bis 4 C-Atomen in der Position von $R^2$ darstellen, wobei die Summe der C-Atome dieser beiden Alkylsubstituenten höchstens 10 beträgt,
b2) Verbindungen der Formel II

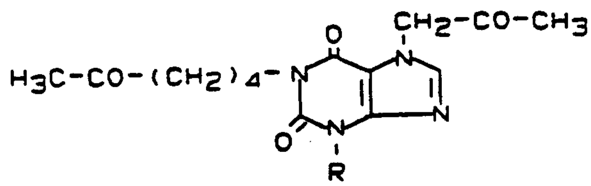

(II)

in der R für einen Alkylrest mit 1 bis 4 C-Atomen steht,
b3) Verbindungen der Formel III

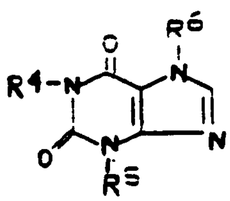

(III),

in der mindestens einer der Reste $R^4$ und $R^6$ eine tertiäre Hydroxyalkylgruppe der Formel

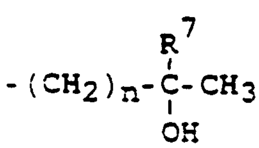

(IIIa)

darstellt, wobei $R^7$ eine Alkylgruppe mit bis zu 3 C-Atomen und n eine ganze Zahl von 2 bis 5 bedeuten, und - falls nur einer der Reste $R^4$ oder $R^6$ eine solche tertiäre Hydroxyalkylgruppe der Formel IIIa bedeutet - der andere Rest für ein Wasserstoffatom oder einen aliphatischen Kohlenwasserstoff-Rest $R^8$ mit bis zu 6 C-Atomen steht, dessen Kohlenstoffkette von bis zu 2 Sauerstoffatomen unterbrochen oder mit einer Oxogruppe oder bis zu zwei Hydroxygruppen substituiert sein kann, und $R^5$ eine Alkylgruppe mit 1 bis 4 C-Atomen darstellt, enthält oder aus diesen Komponenten besteht.

**2.** Kombinationspräparat nach Anspruch 1 dadurch gekennzeichnet, daß die sulfatierten Polysaccharide der Komponente a) ein Molekulargewicht von 1000 bis 200000 Dalton, vorzugsweise von 5000 bis 12000 Dalton besitzen.

**3.** Kombinationspräparat nach einem oder mehreren der Ansprüche 1 bis 2, dadurch gekennzeichnet, daß die sulfatierten Polysaccharide der Komponente a) einen Sulfatierungsgrad der Polysaccharid-OH-Gruppen von 20 bis 100 % vorzugsweise von 50 bis 95 % insbesondere von 90 % besitzen.

4. Kombinationspräparat nach einem oder mehreren der Ansprüche 1 bis 3, dadurch gekennzeichnet, daß die Xanthinderivate der Komponente b) ausgewählt sind aus folgenden Verbindungen:
1-Hexyl-3,7-dimethylxanthin,
1-(5-Hydroxyhexyl)-3,7-dimethylxanthin,
3,7-Dimethyl-1-(5-oxohexyl)-xanthin,
7- (5-Hydroxyhexyl)-1,3-dimethylxanthin,
1,3-Dimethyl-7-(5-oxohexyl)-xanthin,
1-(5-Hydroxyhexyl)-3-methyl-7-propylxanthin,
3-Methyl-1-(5-oxohexyl)-7-propylxanthin und/oder
7-Ethoxymethyl-1-(5-hydroxy-5-methylhexyl)-3-methyl-3-methylxanthin,
insbesondere 3,7-Dimethyl-1-(5-oxohexyl)-xanthin.

5. Kombinationspräparat nach einem oder mehreren der Ansprüche 1 bis 4, dadurch gekennzeichnet, daß das Gewichtsverhältnis der Komponente a) zur Komponente b) zwischen 1:100 und 100:1, vorzugsweise zwischen 1:10 und 30:1 liegt.

6. Kombinationspräparat nach einem oder mehreren der Ansprüche 1 bis 5 als Mittel zur Behandlung bzw. zur Prophylaxe von Infektionskrankheiten, die durch Viren - insbesondere durch Retroviren - verursacht werden.

7. Verwendung von Xanthinderivaten und von sulfatierten Polysacchariden gemäß den Ansprüchen 1 bis 5 zur Herstellung von Arzneimitteln zur Behandlung von durch Viren - insbesondere durch Retroviren - verursachte Krankheiten.

**Patentansprüche für folgende Vertragsstaaten : ES, GR**

1. Verfahren zur Herstellung eines pharmazeutischen Kombinationspräparates, dadurch gekennzeichnet, daß die Komponenten
   a) mindestens ein sulfatiertes Polysaccharid ausgewählt aus Dextranpolysulfat, Laevanpolysulfat und Pentosanpolysulfat, insbesondere Pentosanpolysulfat und
   b) mindestens ein Xanthinderivat der folgenden Verbindungen:
      b1) Verbindungen der Formel I

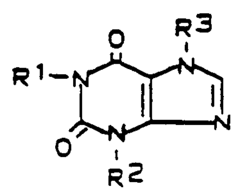

(I)

in der einer der Reste $R^1$ und $R^3$ eine geradkettige Alkyl-, ($\omega$-1)-Oxoalkyl- oder ($\omega$-1)-Hydroxyalkylgruppe mit 3 bis 8 C-Atomen und die beiden anderen Reste $R^2$ und $R^3$ oder $R^1$ und $R^2$ geradkettige oder verzweigte Alkylgruppen mit 1 bis 8 C-Atomen in der Position von $R^1$ und $R^3$ und von 1 bis 4 C-Atomen in der Position von $R^2$ darstellen, wobei die Summe der C-Atome dieser beiden Alkylsubstituenten höchstens 10 beträgt,
b2) Verbindungen der Formel II

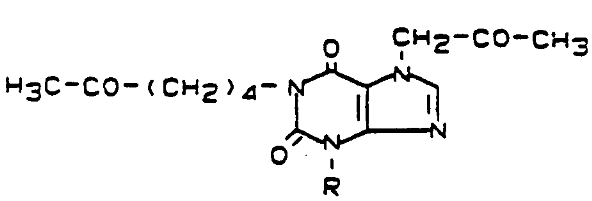

(II)

in der R für einen Alkylrest mit 1 bis 4 C-Atomen steht,
b3) Verbindungen der Formel III

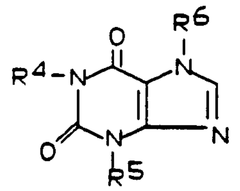

(III),

in der mindestens einer der Reste $R^4$ und $R^6$ eine tertiäre Hydroxyalkylgruppe der Formel

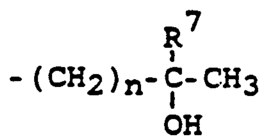

(IIIa)

darstellt, wobei $R^7$ eine Alkylgruppe mit bis zu 3 C-Atomen und n eine ganze Zahl von 2 bis 5 bedeuten, und - falls nur einer der Reste $R^4$ oder $R^6$ eine solche tertiäre Hydroxyalkylgruppe der Formel IIIa bedeutet - der andere Rest für ein Wasserstoffatom oder einen aliphatischen Kohlenwasserstoff-Rest $R^8$ mit bis zu 6 C-Atomen steht, dessen Kohlenstoffkette von bis zu 2 Sauerstoffatomen unterbrochen oder mit einer Oxogruppe oder bis zu zwei Hydroxygruppen substituiert sein kann, und $R^5$ eine Alkylgruppe mit 1 bis 4 C-Atomen darstellt, mit Hilfs- und/oder Trägerstoffen in eine geeignete Darreichungsform gebracht werden.

**2.** Verfahren zur Herstellung eines Kombinationspräparates nach Anspruch 1 dadurch gekennzeichnet, daß die sulfatierten Polysaccharide der Komponente a) ein Molekulargewicht von 1000 bis 200000 Dalton, vorzugsweise von 5000 bis 12000 Dalton besitzen.

**3.** Verfahren zur Herstellung eines Kombinationspräparates nach Anspruch 1 oder 2, dadurch gekennzeichnet, daß die sulfatierten Polysaccharide der Komponente a) einen Sulfatierungsgrad der Polysaccharid-OH-Gruppen von ca. 20 bis 100 % vorzugsweise von ca. 50 bis 95 %, insbesondere von ca. 90 %, besitzen.

**4.** Verfahren zur Herstellung eines Kombinationspräparates nach einem oder mehreren der Ansprüche 1 bis 3, dadurch gekennzeichnet, daß die Xanthinderivate der Komponente b) ausgewählt sind aus folgenden Verbindungen:
1-Hexyl-3,7-dimethylxanthin,
1-(5-Hydroxyhexyl)-3,7-dimethylxanthin,
3,7-Dimethyl-1-(5-oxohexyl)-xanthin,
7-(5-Hydroxyhexyl)-1,3-dimethylxanthin,
1,3-Dimethyl-7-(5-oxohexyl)-xanthin,
1-(5-Hydroxyhexyl)-3-methyl-7-propylxanthin,
3-Methyl-1-(5-oxohexyl)-7-propylxanthin und/oder
7-Ethoxymethyl-1-(5-hydroxy-5-methylhexyl)-3-methylxanthin,
insbesondere 3,7-Dimethyl-1-(5-oxohexyl)-xanthin.

**5.** Verfahren zur Herstellung eines Kombinationspräparates nach Anspruch 1, dadurch gekennzeichnet, daß das Gewichtsverhältnis der Komponente a) zur Komponente b) zwischen 1:100 und 100:1, vorzugsweise zwischen 1:10 und 30:1 liegt.

## Claims

**Claims for the following Contracting States : AT, BE, CH, DE, FR, GB, IT, LI, LU, NL, SE**

**1.** A pharmaceutical combination product, which contains the components

a) at least one sulfated polysaccharide selected from dextran polysulfate, levan polysulfate and pentosan polysulfate, especially pentosan polysulfate and

b) at least one xanthine derivative of the following compounds:

b1) compounds of the formula I

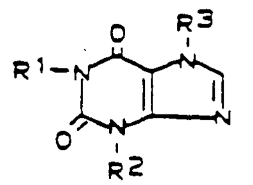

(I)

in which one of the radicals $R^1$ and $R^3$ represents a straight-chain alkyl, ($\omega$-1)-oxoalkyl or ($\omega$-1)-hydroxyalkyl group having 3 to 8 carbon atoms, and the two other radicals $R^2$ and $R^3$ or $R^1$ and $R^2$ represent straight-chain or branched alkyl groups having 1 to 8 carbon atoms in the position of $R^1$ and $R^3$ and 1 to 4 carbon atoms in the position of $R^2$, with the total of carbon atoms in these two alkyl substituents being not more than 10,

b2) compounds of the formula II

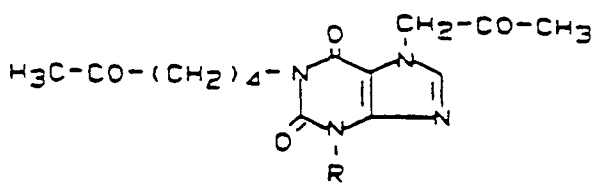

(II)

in which R represents an alkyl radical having 1 to 4 carbon atoms,

b3) compounds of the formula III

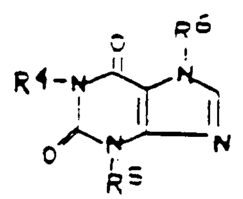

(III)

in which at least one of the radicals $R^4$ and $R^6$ represents a tertiary hydroxyalkyl group of the formula

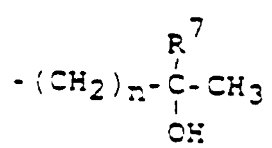

(IIIa)

where $R^7$ denotes an alkyl group having up to 3 carbon atoms and n denotes an integer from 2 to 5, and - if only one of the radicals $R^4$ or $R^6$ denotes such a tertiary hydroxyalkyl group of the formula IIIa - the other radical represents a hydrogen atom or an aliphatic hydrocarbon radical $R^8$ which has up to 6 carbon atoms and whose carbon chain can be interrupted by up to 2 oxygen

atoms or substituted by an oxo group or up to two hydroxyl groups, and $R^5$ represents an alkyl group having 1 to 4 carbon atoms,
or which is composed of these components.

**2.** A combination product as claimed in claim 1, wherein the sulfated polysaccharides of component a) have a molecular weight of 1,000 to 200,000 Dalton, preferably of 5,000 to 12,000 Dalton.

**3.** A combination product as claimed in one or both of claims 1 and 2, wherein the sulfated polysaccharides of component a) have a degree of sulfation of the polysaccharide OH groups of 20 to 100%, preferably of 50 to 95%, especially of 90%.

**4.** A combination product as claimed in one or more of claims 1 to 3, wherein the xanthine derivatives of component b) are selected from the following compounds:
1-hexyl-3,7-dimethylxanthine,
1-(5-hydroxyhexyl)-3,7-dimethylxanthine,
3,7-dimethyl-1-(5-oxohexyl)xanthine,
7-(5-hydroxyhexyl)-1,3-dimethylxanthine,
1,3-dimethyl-7-(5-oxohexyl)xanthine,
1-(5-hydroxyhexyl)-3-methyl-7-propylxanthine,
3-methyl-1-(5-oxohexyl)-7-propylxanthine and/or
7-ethoxymethyl-1-(5-hydroxy-5-methylhexyl)-3-methylxanthine, especially 3,7-dimethyl-1-(5-oxohexyl)-xanthine.

**5.** A combination product as claimed in one or more of claims 1 to 4, wherein the ratio by weight of component a) to component b) is between 1:100 and 100:1, preferably between 1:10 and 30:1.

**6.** A combination product as claimed in one or more of claims 1 to 5 as an agent for the treatment or for the prophylaxis of infectious diseases caused by viruses - especially by retroviruses.

**7.** The use of xanthine derivatives and of sulfated polysaccharides as claimed in claims 1 to 5 for the preparation of medicaments for the treatment of diseases caused by viruses - especially by retroviruses.

**Claims for the following Contracting States : ES, GR**

**1.** A process for the preparation of a pharmaceutical combination product, which comprises converting the components

a) at least one sulfated polysaccharide selected from dextran polysulfate, levan polysulfate and pentosan polysulfate, especially pentosan polysulfate and
b) at least one xanthine derivative of the following compounds:
b1) compounds of the formula I

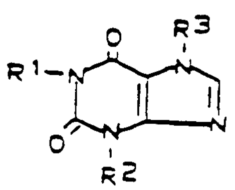

(I)

in which one of the radicals $R^1$ and $R^3$ represents a straight-chain alkyl, ($\omega$-1)-oxoalkyl or ($\omega$-1)-hydroxyalkyl group having 3 to 8 carbon atoms, and the two other radicals $R^2$ and $R^3$ or $R^1$ and $R^2$ represent straight-chain or branched alkyl groups having 1 to 8 carbon atoms in the position of $R^1$ and $R^3$ and 1 to 4 carbon atoms in the position of $R^2$, with the total of carbon atoms in these two alkyl substituents being not more than 10,
b2) compounds of the formula II

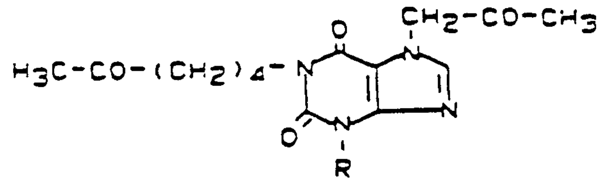

(II)

in which R represents an alkyl radical having 1 to 4 carbon atoms,
b3) compounds of the formula III

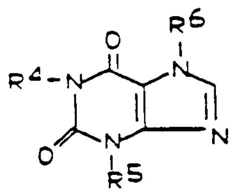

(III)

in which at least one of the radicals $R^4$ and $R^6$ represents a tertiary hydroxyalkyl group of the formula

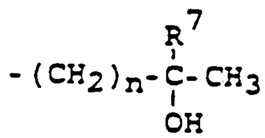

(IIIa)

where $R^7$ denotes an alkyl group having up to 3 carbon atoms and n denotes an integer from 2 to 5, and - if only one of the radicals $R^4$ or $R^6$ denotes such a tertiary hydroxyalkyl group of the formula IIIa - the other radical represents a hydrogen atom or an aliphatic hydrocarbon radical $R^8$ which has up to 6 carbon atoms and whose carbon chain can be interrupted by up to 2 oxygen atoms or substituted by an oxo group or up to two hydroxyl groups, and $R^5$ represents an alkyl group having 1 to 4 carbon atoms, into a suitable administration form with auxiliaries and/or vehicles.

**2.** The process for the preparation of a combination product as claimed in claim 1, wherein the sulfated polysaccharides of component a) have a molecular weight of 1,000 to 200,000 Dalton, preferably of 5,000 to 12,000 Dalton.

**3.** The process for the preparation of a combination product as claimed in claim 1 or 2, wherein the sulfated polysaccharides of component a) have a degree of sulfation of the polysaccharide OH groups of about 20 to 100%, preferably of about 50 to 95%, especially of about 90%.

**4.** The process for the preparation of a combination product as claimed in one or more of claims 1 to 3, wherein the xanthine derivatives of component b) are selected from the following compounds:
1-hexyl-3,7-dimethylxanthine,
1-(5-hydroxyhexyl)-3,7-dimethylxanthine,
3,7-dimethyl-1-(5-oxohexyl)xanthine,
7-(5-hydroxyhexyl)-1,3-dimethylxanthine,
1,3-dimethyl-7-(5-oxohexyl)xanthine,
1-(5-hydroxyhexyl)-3-methyl-7-propylxanthine,
3-methyl-1-(5-oxohexyl)-7-propylxanthine and/or
7-ethoxymethyl-1-(5-hydroxy-5-methylhexyl)-3-methylxanthine, especially 3,7-dimethyl-1-(5-oxohexyl)-xanthine.

**5.** The process for the preparation of a combination product as claimed in claim 1, wherein the ratio by weight of component a) to component b) is between 1:100 and 100:1, preferably between 1:10 and 30:1.

**Revendications**

**Revendications pour les Etats contractants suivants : AT, BE, CH, DE, FR, GB, IT, LI, LU, NL, SE**

**1.** Préparation pharmaceutique à base d'une association, caractérisée en ce qu'elle contient les composants suivants ou elle est constituée des composants suivants:

a) au moins un polysaccharide sulfaté choisi parmi le polysulfate de dextrane, le polysulfate de lévane et le polysulfate de pentosane, en particulier le polysulfate de pentosane, et

b) au moins un dérivé xanthique des composés suivants:

b1) composés de formule I

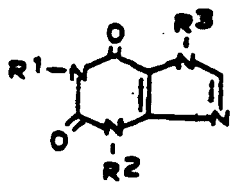

(I)

dans laquelle un des radicaux $R^1$ et $R^3$ représente un groupe alkyle, ($\omega$-1)-oxoalkyle ou ($\omega$-1)-hydroxyalkyle, à chaîne droite, comportant 3 à 8 atomes de carbone, et les deux autres radicaux, $R^2$ et $R^3$ ou $R^1$ et $R^2$, représentent des groupes alkyle, à chaîne droite ou ramifiée, comportant 1 à 8 atomes de carbone dans la position de $R^1$ et $R^3$, et 1 à 4 atomes de carbone dans la position de $R^2$, la somme des atomes de carbone de ces deux substituants alkyle étant au plus égale à 10,

b2) composés de formule II

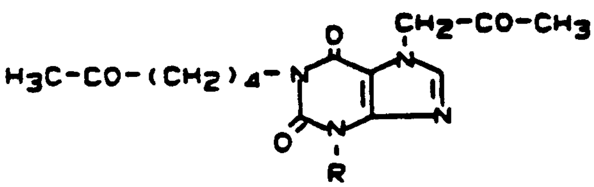

(II)

dans laquelle R représente un radical alkyle comportant 1 à 4 atomes de carbone,

b3) composés de formule III

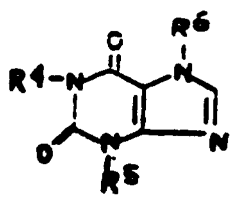

(III)

dans laquelle au moins un des radicaux $R^4$ et $R^6$ représente un groupe hydroxyalkyle tertiaire de formule:

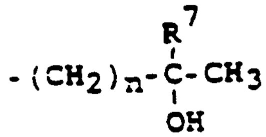 (IIIa)

$R^7$ représentant un groupe alkyle comportant jusqu'à 3 atomes de carbone et n étant un nombre entier valant de 2 à 5, et - dans le cas ou un seul des radicaux $R^4$ ou $R^6$ représente un tel groupe hydroxyalkyle tertiaire de formule (IIIa) -, l'autre radical représente un atome d'hydrogène ou un radical hydrocarboné aliphatique $R^8$ comportant jusqu'à 6 atomes de carbone, la chaîne carbonée de celui-ci pouvant être interrompue par jusqu'à 2 atomes d'oxygène ou pouvant être substituée par un groupe oxo ou par jusqu'à deux groupes hydroxy, et $R^5$ représente un groupe alkyle comportant 1 à 4 atomes de carbone.

**2.** Préparation à base d'une association selon la revendication 1, caractérisée en ce que les polysaccharides sulfatés du composant a) présentent une masse moléculaire de 1000 à 200000 Dalton, de préférence de 5000 à 12000 Dalton.

**3.** Préparation à base d'une association selon une ou plusieurs des revendications 1 à 2, caractérisée en ce que les polysaccharides sulfatés du composant a) présentent un degré de sulfatation des groupes OH de polysaccharides allant de 20 à 100%, de préférence de 50 à 95%, en particulier de 90%.

**4.** Préparation à base d'une association selon une ou plusieurs des revendications 1 à 3, caractérisée en ce que les dérivés xanthiques du composant b) sont choisis parmi les composés suivants:
1-hexyl-3,7-diméthylxanthine,
1-(5-hydroxyhexyl)-3,7-diméthylxanthine,
3,7-diméthyl-1-(5-oxohexyl)-xanthine,
7-(5-hydroxyhexyl)-1,3-diméthylxanthine,
1,3-diméthyl-7-(5-oxohexyl)-xanthine,
1-(5-hydroxyhexyl)-3-méthyl-7-propylxanthine,
3-méthyl-1-(5-oxohexyl)-7-propylxanthine, et/ou
7-éthoxyméthyl-1-(5-hydroxy-5-méthylhexyl)-3-méthylxanthine, en particulier 3,7-diméthyl-1-(5-oxohexyl)-xanthine.

**5.** Préparation à base d'une association selon une ou plusieurs des revendications 1 à 4, caractérisée en ce que le rapport du composant a) au composant b) est compris, en poids, entre 1:100 et 100:1, de préférence entre 1:10 et 30:1.

**6.** Préparation à base d'une association selon une ou plusieurs des revendications 1 à 5, en tant qu'agent destiné au traitement ou bien à la prophylaxie de maladies infectieuses provoquées par des virus, en particulier par des rétrovirus.

**7.** Utilisation de dérivés xanthiques et de polysaccharides sulfates selon les revendications 1 à 5 pour préparer des médicaments destinés au traitement de maladies provoquées par des virus, en particulier par des rétrovirus.

**Revendications pour les Etats contractants suivants : ES, GR**

**1.** Procédé pour fabriquer une préparation pharmaceutique à base d'une association, caractérisé en ce qu'on met sous une forme de présentation appropriée, avec des adjuvants et/ou des véhicules, les composants suivants:
a) au moins un polysaccharide sulfaté choisi parmi le polysulfate de dextrane, le polysulfate de lévane et le polysulfate de pentosane, en particulier le polysulfate de pentosane, et
b) au moins un dérivé xanthique des composés suivants:
b1) composés de formule I

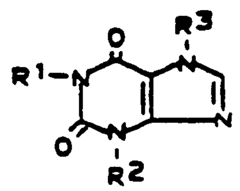

(I)

dans laquelle un des radicaux $R^1$ et $R^3$ représente un groupe alkyle, ($\omega$-1)-oxoalkyle ou ($\omega$-1)-hydroxyalkyle, à chaîne droite, comportant 3 à 8 atomes de carbone, et les deux autres radicaux, $R^2$ et $R^3$ ou $R^1$ et $R^2$, représentent des groupes alkyle, à chaîne droite ou ramifiée, comportant 1 à 8 atomes de carbone dans la position de $R^1$ et $R^3$, et 1 à 4 atomes de carbone dans la position de $R^2$, la somme des atomes de carbone de ces deux substituants alkyle étant au plus égale à 10,
b2) composés de formule II

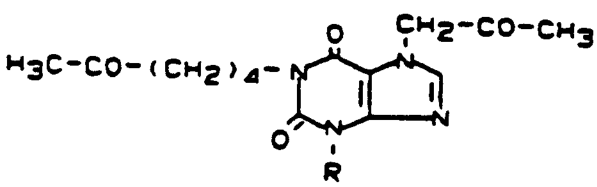

(II)

dans laquelle R représente un radical alkyle comportant 1 à 4 atomes de carbone,
b3) composés de formule III

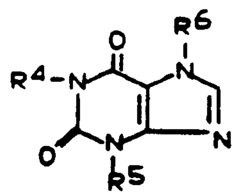

(III)

dans laquelle au moins un des radicaux $R^4$ et $R^6$ représente un groupe hydroxyalkyle tertiaire de formule:

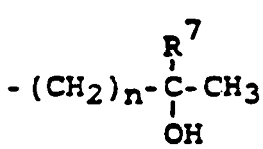

(IIIa)

$R^7$ représentant un groupe alkyle comportant jusqu'à 3 atomes de carbone et n étant un nombre entier valent de 2 à 5, et - dans le cas ou un seul des radicaux $R^4$ ou $R^6$ représente un tel groupe hydroxyalkyle tertiaire de formule (IIIa) -, l'autre radical représente un atome d'hydrogène ou un radical hydrocarboné aliphatique $R^8$ comportant jusqu'à 6 atomes de carbone, la chaîne carbonée de celui-ci pouvant être interrompue par jusqu'à 2 atomes d'oxygène ou pouvant être substituée par un groupe oxo ou par jusqu'à deux groupes hydroxy, et $R^5$ représente un groupe alkyle comportant 1 à 4 atomes de carbone.

**2.** Procédé pour fabriquer une préparation à base d'une association selon la revendication 1, caractérisé en ce que les polysaccharides sulfatés du composent a) présentent une masse moléculaire de 1000 à

200000 Dalton, de préférence de 5000 à 12000 Dalton.

3. Procédé pour fabriquer une préparation à base d'une association la revendication 1 ou 2, caractérisé en ce que les polysaccharides sulfatés du composent a) présentent un degré de sulfatation des groupes OH de polysaccharides allant d'environ 20 à 100%, de préférence d'environ 50 à 95%, en particulier d'environ 90%.

4. Procédé pour fabriquer une préparation à base d'une association selon une ou plusieurs des revendications 1 à 3, caractérisé en ce que les dérivés xanthiques du composant b) sont choisis parmi les composés suivants:
1-hexyl-3,7-diméthylxanthine,
1-(5-hydroxyhexyl)-3,7-diméthylxanthine,
3,7-diméthyl-1-(5-oxohexyl)-xanthine,
7-(5-hydroxyhexyl)-1,3-diméthylxanthine,
1,3-diméthyl-7-(5-oxohexyl)-xanthine,
1-(5-hydroxyhexyl)-3-méthyl-7-propylxanthine,
3-méthyl-1-(5-oxohexyl)-7-propylxanthine, et/ou
7-éthoxyméthyl-1-(5-hydroxy-5-méthylhexyl)-3-méthylxanthine, en particulier 3,7-diméthyl-1-(5-oxohexyl)-xanthine.

5. Procédé pour fabriquer une préparation à base d'une association selon la revendication 1, caractérisé en ce que le rapport du composant a) au composant b) est compris, en poids, entre 1:100 et 100:1, de préférence entre 1:10 et 30:1.